# EUROPEAN PATENT SPECIFICATION

(11) **EP 4 501 244 B1**
(45) Date of publication and mention of the grant of the patent: **01.07.2026**
(21) Application number: 23780796.1
(22) Date of filing: 29.03.2023
(51) Int. Cl.: A61B 8/00, A61B 6/00, A61B 6/04

(54) **COMPRESSION MEMBER AND IMAGE ACQUISITION METHOD**
KOMPRESSIONSELEMENT UND BILDAUFNAHMEVERFAHREN
ÉLÉMENT DE COMPRESSION ET PROCÉDÉ D'ACQUISITION D'IMAGES

(30) Priority: 29.03.2022 JP 2022054511
(43) Date of publication of application: 05.02.2025
(73) Proprietor: FUJIFILM Corporation, Tokyo 106-8620 (JP)
(72) Inventor: OSAWA, Atsushi, Ashigarakami-gun, Kanagawa 258-8538 (JP)
(74) Representative: Dehns Germany Partnerschaft mbB
(86) International application number: PCT/JP2023/013019
(87) International publication number: WO 2023/190788

(56) References cited:
- JP-A- 2009 082 399
- JP-A- 2011 212 111
- JP-A- 2011 212 111
- JP-A- 2017 153 571
- JP-A- 2018 064 833
- JP-A- 2020 162 931
- US-A- 5 833 627
- US-A- 5 851 180
- US-A1- 2018 110 484
- US-A1- 2020 228 092
- RODRIGUEZ-MOLARES ALFONSO ET AL: "Specular Beamforming", IEEE TRANSACTIONS ON ULTRASONICS, FERROELECTRICS, AND FREQUENCY CONTROL, IEEE, USA, vol. 64, no. 9, 1 September 2017 (2017-09-01), pages 1285 - 1297, XP011659657, ISSN: 0885-3010, [retrieved on 20170828], DOI: 10.1109/TUFFC.2017.2709038

## Description

### BACKGROUND OF THE INVENTION

### 1. Field of the Invention

The present disclosure relates to a compression member, an image acquisition device, and an image acquisition method.

### 2. Description of the Related Art

In the related art, an image acquisition device that captures a radiation image of a breast is known. In addition, from the viewpoint of improving a detection accuracy of breast cancer, a device capable of acquiring an ultrasound image of a breast in addition to a radiation image has been proposed. For example, JP2005-125080A discloses acquiring an X-ray image of a breast, determining coordinates of an ROI that can be observed on an X-ray image and includes an abnormal portion, and scanning an object with an ultrasound imaging system by using the coordinates of the ROI.

In addition, it is well known that the breast is compressed with a compression member in the capturing of the radiation image by the image acquisition device, but a method of compressing the breast with the compression member has been proposed also in a case of acquiring the ultrasound image. For example, JP2021-527519A discloses that a pair of compression paddles having a patient contact surface that accommodates a transducer and comprises a compression material for fixing a breast for ultrasound imaging is moved by a motor. In addition, it has been disclosed that a patient contact surface of the compression paddle is in a stripe pattern, is provided with a groove, or is roughened in a different manner to resist movement of breast tissue caused by sweat, a coupling gel required for the ultrasound imaging, and the like. JP 2011 212111 A relates to a compression paddle and an X-ray imaging apparatus. JP 2009 082399 A relates to a medical imaging apparatus and a medical imaging method.

### SUMMARY OF THE DISCLOSURE

The invention is set out in the appended set of claims.

Meanwhile, it is known that an image quality of an ultrasound image depends on a difference in acoustic impedance of each medium through which an ultrasound wave is transmitted. At an interface in each medium, since the smaller the difference in acoustic impedance is, the more ultrasound waves are transmitted, the signal strength is increased and the image quality is improved, and since the larger the difference in acoustic impedance is, the more ultrasound waves are reflected, noise is increased and the image quality deteriorates.

In a case where an ultrasound image of the breast put into a compressed state by a compression member is acquired, the ultrasound wave is transmitted through the compression member, the breast, and the like as a medium. In the compression member that has been used in the related art, there is a case where a difference in acoustic impedance with the breast is large and the image quality of the obtained ultrasound image is poor.

The present disclosure provides a compression member, an image acquisition system, and an image acquisition method capable of obtaining a favorable image.

A compression member is provided as defined by claim 1.

According to an embodiment, the uneven structure may be provided in at least a part of a region on at least the contact surface.

According to an embodiment, the uneven structure may be provided in at least a part of a region on each of the contact surface and the outer surface.

According to an embodiment, at least one of a shape of unevenness, a maximum height roughness, or an average length may be different between the uneven structure provided on the contact surface and the uneven structure provided on the outer surface.

The maximum height roughness and the average length of the uneven structure may be equal to or less than 1/10 of the wavelength of the ultrasound wave.

According to an embodiment, the maximum height roughness and the average length of the uneven structure may be equal to or less than 1/20 of the wavelength of the ultrasound wave.

An image acquisition system is provided as defined in claim 4.

According to an embodiment, the maximum height roughness (Rz) and the average length (Rsm) of the uneven structure may be equal to or less than 1/20 of the wavelength of the ultrasound wave.

An image acquisition method is provided as defined by claim 6.

According to an embodiment, the medium may have acoustic impedance between acoustic impedance of the breast and acoustic impedance of the compression member.

According to an embodiment, , a concave portion of the uneven structure may be filled with the medium.

According to the above-described aspects and embodiments, the compression member, the image acquisition system, and the image acquisition method according to the present disclosure can obtain a favorable image.

### BRIEF DESCRIPTION OF THE DRAWINGS

Fig. 1 is a diagram showing an example of a schematic configuration of an image acquisition system.
Fig. 2 is a side view showing an example of an appearance of the image acquisition device.
Fig. 3 is a three-view showing an example of a schematic configuration of a compression member.
Fig. 4 is an enlarged cross-sectional view showing an example of an uneven structure of the compression member.
Fig. 5 is an enlarged cross-sectional view showing an example of the uneven structure of the compression member.
Fig. 6 is an enlarged cross-sectional view showing an example of the uneven structure of the compression member.
Fig. 7 is an enlarged cross-sectional view showing an example of the uneven structure of the compression member.
Fig. 8 is an enlarged cross-sectional view showing an example of the uneven structure of the compression member.
Fig. 9 is an enlarged cross-sectional view showing an example of the uneven structure of the compression member.
Fig. 10 is a block diagram showing an example of a hardware configuration of a console.
Fig. 11 is a block diagram showing an example of a functional configuration of the console.
Fig. 12 is a flowchart showing an example of a control process of the console.
Fig. 13 is a flowchart showing an example of an image acquisition method.
Fig. 14 is an enlarged cross-sectional view showing an example of a compression member according to a comparative example.
Fig. 15 is a three-view showing an example of a schematic configuration of the compression member.

### DESCRIPTION OF THE PREFERRED EMBODIMENTS

Hereinafter, a description of embodiments of the present disclosure will be made with reference to the accompanying drawings.

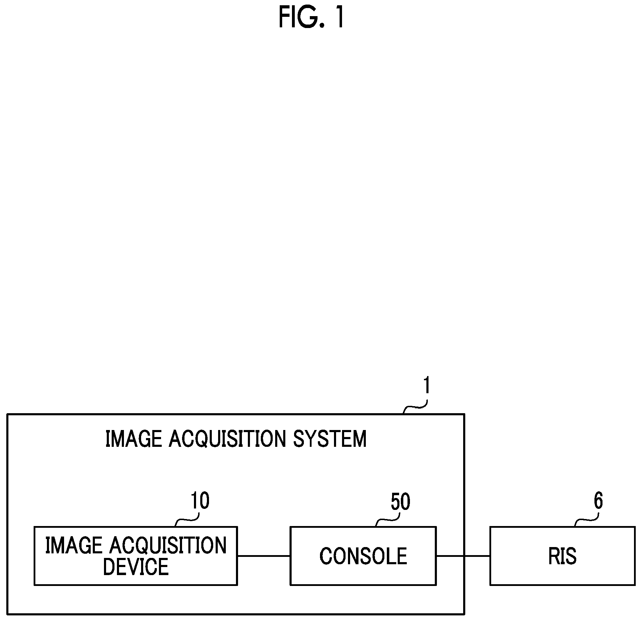

First, a configuration of an image acquisition system 1 to which an image acquisition device 10 of the present disclosure is applied will be described with reference to Fig. 1. Fig. 1 is a diagram showing a schematic configuration of the image acquisition system 1. As shown in Fig. 1, the image acquisition system 1 comprises the image acquisition device 10 and a console 50. The image acquisition device 10 and the console 50, and the console 50 and an external radiology information system (RIS) 6 are configured to be connectable to each other via a wired or wireless network.

In the image acquisition system 1, the console 50 acquires an imaging order or the like from the RIS 6 and controls the image acquisition device 10 in accordance with the imaging order, an instruction from the user, and the like. The image acquisition device 10 acquires a radiation image and an ultrasound image of a breast of a subject put into a compressed state by a compression member 40 as a subject.

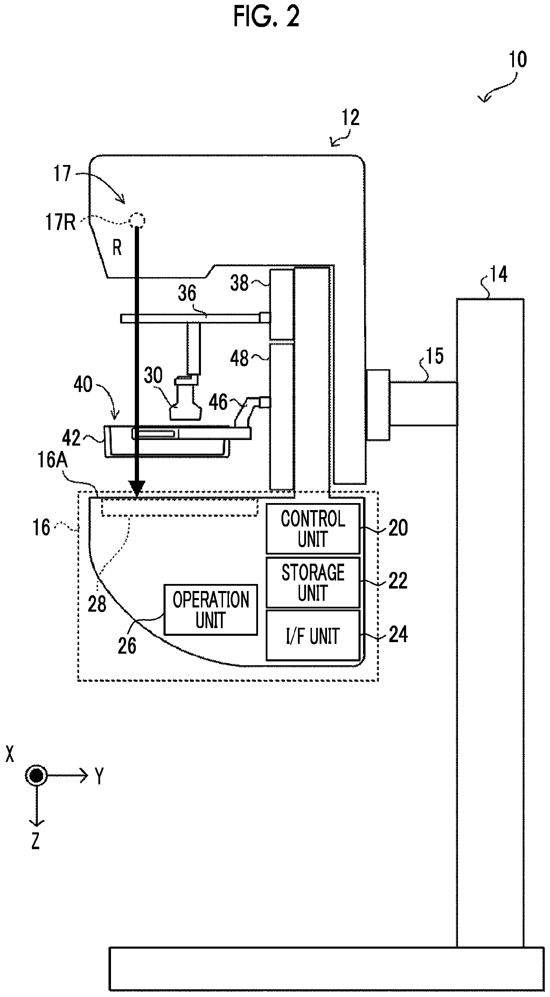

Next, a schematic configuration of the image acquisition device 10 will be described with reference to Fig. 2. Fig. 2 is a side view showing an example of an appearance of the image acquisition device 10 and is a view in a case where the image acquisition device 10 is viewed from the right side of a subject. As shown in Fig. 2, the image acquisition device 10 comprises a radiation source 17R, a radiation detector 28, an imaging table 16 disposed between the radiation source 17R and the radiation detector 28, the compression member 40 that compresses the breast between the compression member 40 and the imaging table 16, and an ultrasound probe 30. In the image acquisition device 10, a user such as a doctor or a technician positions the breast of the subject on an imaging surface 16A of the imaging table 16.

The image acquisition device 10 comprises an arm part 12, a base 14, and a shaft part 15. The arm part 12 is movably held in the vertical direction (Z direction) by the base 14. The shaft part 15 connects the arm part 12 to the base 14. The arm part 12 is rotatable relative to the base 14 with the shaft part 15 as a rotation axis. In addition, the arm part 12 may be relatively rotatable with respect to the base 14 with the shaft part 15 as the rotation axis separately between an upper part comprising a radiation emitting unit 17 and a lower part comprising the imaging table 16.

The arm part 12 comprises the radiation emitting unit 17 and the imaging table 16. The radiation emitting unit 17 comprises the radiation source 17R and is configured to change an irradiation field of radiation (for example, X-rays) emitted from the radiation source 17R. For example, the change of the irradiation field may be performed by the user operating an operation unit 26 or may be performed by a control unit 20 in accordance with a type of the attached compression member 40.

The imaging table 16 comprises the control unit 20, a storage unit 22, an interface (I/F) unit 24, the operation unit 26, and the radiation detector 28. The control unit 20 controls an overall operation of the image acquisition device 10 in accordance with the control of the console 50. The control unit 20 comprises a central processing unit (CPU), a read only memory (ROM), a random access memory (RAM), and the like (not shown). The ROM stores in advance various programs including a program executed by the CPU for performing control regarding acquisition of the radiation image and the ultrasound image. The RAM transitorily stores various types of data.

Data of the radiation image and the ultrasound image, various types of other information, and the like are stored in the storage unit 22. The storage unit 22 is realized by, for example, a storage medium, such as a hard disk drive (HDD), a solid state drive (SSD), and a flash memory.

The I/F unit 24 communicates various types of information with the console 50 by wired communication or wireless communication. Specifically, the I/F unit 24 receives information regarding the control of the image acquisition device 10 from the console 50. In addition, the I/F unit 24 transmits the data of the radiation image and the ultrasound image to the console 50.

The operation unit 26 is a part provided on the imaging table 16 or the like and operable by the user with a hand, a foot, or the like, and is, for example, a switch, a button, a touch panel, or the like.

The radiation detector 28 is disposed inside the imaging table 16, detects the radiation R transmitted through the breast and the imaging table 16, generates the radiation image based on the detected radiation R, and outputs image data indicating the generated radiation image. It should be noted that a type of the radiation detector 28 is not particularly limited and may be, for example, an indirect conversion type radiation detector that converts the radiation R into light and converts the converted light into a charge, or may be a direct conversion type radiation detector that directly converts the radiation R into a charge.

Further, a probe unit 38 and a compression unit 48 are connected to the arm part 12. A support part 36 that attachably and detachably supports the ultrasound probe 30 is attached to the probe unit 38. The support part 36 (ultrasound probe 30) is moved in the vertical direction and the horizontal direction (X direction, Y direction, and Z direction) by a driving unit (not shown) provided in the probe unit 38. The support part 36 is preferably formed of a material that transmits the radiation R.

The ultrasound probe 30 is used for obtaining the ultrasound image of the breast put into the compressed state by the compression member 40, is disposed between the radiation source 17R and the compression member 40, irradiates the breast with ultrasound waves via the compression member 40, and receives reflected waves from the breast. Specifically, the ultrasound probe 30 comprises an ultrasound transducer array. The ultrasound transducer array has a configuration in which a plurality of ultrasound transducers are arranged one-dimensionally or two-dimensionally. The ultrasound transducer is formed, for example, such that electrodes are formed on both ends of a piezoelectric body, such as a piezoelectric ceramic represented by lead (Pb) zirconate titanate (PZT) or a polymer piezoelectric element represented by polyvinylidene difluoride (PVDF). Further, the probe unit 38 includes a converter (not shown) that converts the reflected waves from the breast received by the ultrasound probe 30 into the ultrasound image, and the ultrasound image is obtained by the converter.

In addition, a plurality of different types of ultrasound probes 30 may be interchangeably attached to the image acquisition device 10. Specifically, in accordance with a physique of the subject (for example, a size of the breast), a tissue composition of the breast (for example, a fat mass and a mammary gland mass), a type of imaging (for example, magnified imaging and spot imaging), and the like, the ultrasound probes 30 each having different performances and dimensions may be attached. For example, a linear probe having a center frequency of about 7.5 MHz (for superficial use, or the like), a convex probe having a center frequency of about 3.5 MHz (for abdomen, or the like), a sector probe having a center frequency of about 2.5 MHz (for heart, or the like), and the like may be used.

A support part 46 that supports the compression member 40 is attachably and detachably attached to the compression unit 48. The support part 46 (compression member 40) is moved in the vertical direction (Z direction) by a driving unit (not shown) provided in the compression unit 48.

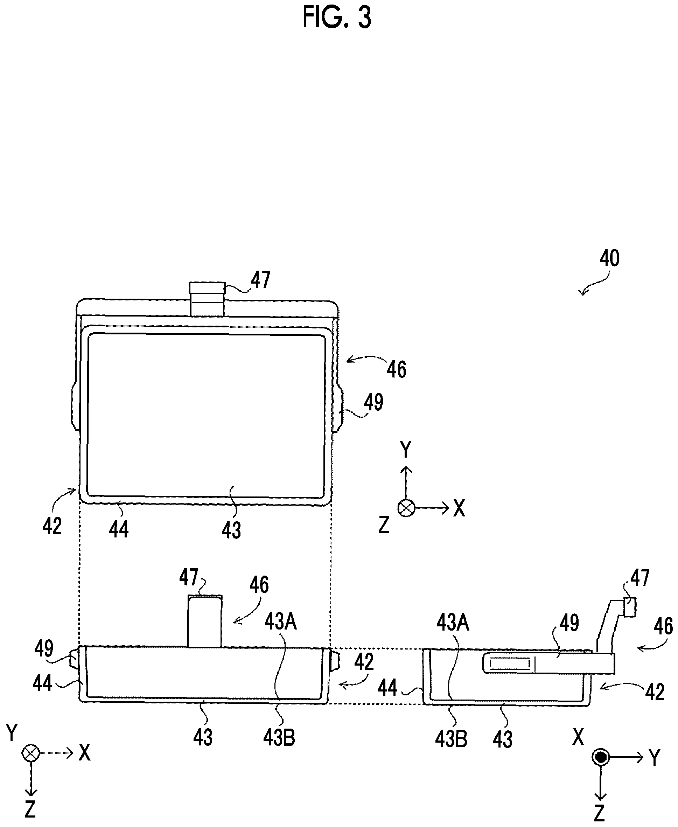

The compression member 40 is disposed between the radiation source 17R and the imaging table 16 and sandwiches the breast between the compression member 40 and the imaging table 16 to put the breast into a compressed state. Fig. 3 shows a three-view of an example of the compression member 40. The three-view of Fig. 3 includes a top view of the compression member 40 as viewed from above (radiation emitting unit 17 side), a side view thereof as viewed from the subject side, and a side view thereof as viewed from the right side of the subject. As shown in Fig. 3, the compression member 40 includes a compression part 42 and the support part 46.

The support part 46 includes an attachment portion 47 and an arm 49. The attachment portion 47 attaches the compression member 40 to the image acquisition device 10, specifically, the driving unit of the compression unit 48. The arm 49 supports the compression part 42.

In the compression part 42, a bottom portion 43 is surrounded by a wall portion 44 having a substantially uniform height, and a cross-sectional shape is formed in a concave shape. The compression part 42 is preferably formed of an optically transparent or translucent material in order to perform positioning and confirmation of a compressed state in compression of the breast. In addition, the compression part 42 is preferably formed of a material excellent in transmittance of the radiation R and ultrasound waves. Further, the compression part 42 is preferably formed of, for example, a material excellent in strength such as drop strength and compression strength.

As such a material, for example, a resin such as polymethylpentene (PMP), polycarbonate (PC), acryl, polypropylene (PP), and polyethylene terephthalate (PET) can be used. In particular, in the polymethylpentene, acoustic impedance, which affects transmissivity and reflectivity of ultrasound waves, is close to that of a human body (breast) as compared with other materials, and a proportion of noise on the ultrasound image can be reduced. Therefore, the polymethylpentene is suitable as the material for the compression part 42.

The bottom portion 43 is configured to be substantially flat, and has an uneven structure in at least a part of a region on at least one of a contact surface 43B with the breast or an upper surface 43A that is an outer surface facing the contact surface 43B and is on the radiation emitting unit 17 side. The uneven structure may be, for example, a structure in which a depth in the Z direction from the surface seems to change in one of the X direction or the Y direction of Fig. 3, such as a stripe shape. In addition, for example, a structure in which the depth in the Z direction from the surface seems to change in both the X direction and the Y direction of Fig. 3, such as a lattice shape and a honeycomb structure shape, may be used.

A maximum height roughness Rz and an average length RSm of the uneven structure are smaller than the wavelength of the ultrasound wave emitted by the ultrasound probe 30. By making the maximum height roughness Rz and the average length Rsm of the uneven structure smaller than the wavelength of the ultrasound wave, an appropriate amount of an echo jelly 4 can be held without affecting the ultrasound image (details will be described below). Specifically, the maximum height roughness Rz and the average length RSm of the uneven structure are preferably equal to or less than 1/10 of the wavelength of the ultrasound wave, and more preferably equal to or less than 1/20 of the wavelength of the ultrasound wave.

For example, in a case where a linear probe (for superficial use, or the like) having a center frequency of about 7.5 MHz is applied as the ultrasound probe 30, an average wavelength of the ultrasound wave transmitted through the compression member 40 and the breast is estimated to be about 200 µm. In this case, the maximum height roughness Rz and the average length Rsm of the uneven structure of the contact surface 43B and/or the upper surface 43A are preferably equal to or less than 20 µm, and more preferably equal to or less than 10 µm.

In addition, for example, in a case where a convex probe (for abdomen or the like) having a center frequency of about 3.5 MHz is applied as the ultrasound probe 30, the average wavelength of the ultrasound wave transmitted through the compression member 40 and the breast is estimated to be about 430 µm. In this case, the maximum height roughness Rz and the average length Rsm of the uneven structure of the contact surface 43B and/or the upper surface 43A are preferably equal to or less than 43 µm, and more preferably equal to or less than 21 µm.

In addition, for example, in a case where a sector probe (for heart or the like) having a center frequency of about 2.5 MHz is applied as the ultrasound probe 30, the average wavelength of the ultrasound waves transmitted through the compression member 40 and the breast is estimated to be about 600 µm. In this case, the maximum height roughness Rz and the average length Rsm of the uneven structure of the contact surface 43B and/or the upper surface 43A are preferably equal to or less than 60 µm, and more preferably equal to or less than 30 µm.

In addition, in a case where the uneven structure is too fine, the transparency of the compression member 40 is lost, and the breast is not visible through the compression member 40, so that the uneven structure is preferably rough to the extent that the positioning of the breast is not impaired. Specifically, it is more preferable that the maximum height roughness Rz and the average length RSm of the uneven structure of the contact surface 43B and/or the upper surface 43A are equal to or more than 5 µm.

In addition, it is preferable that an uneven structure is provided in at least a part of a region on at least the contact surface 43B, and it is more preferable that an uneven structure is provided in at least a part of a region on each (both side) of the contact surface 43B and the upper surface 43A. In addition, in a case where each of the contact surface 43B and the upper surface 43A has the uneven structure, at least one of a shape of unevenness, the maximum height roughness Rz, or the average length RSm may be different between the uneven structure provided on the contact surface 43B and the uneven structure provided in the upper surface 43A.

In addition, it is preferable that at least one of a region of the contact surface 43B with which the breast may be in contact or a region of the upper surface 43A facing the region has an uneven structure. That is, for example, the uneven structure may not be provided in a region of an outer edge portion of the contact surface 43B, in which a possibility that the breast comes into contact is low, and the region of the upper surface 43A facing the region.

A method of forming an uneven structure is not particularly limited, and for example, a known method such as a mechanical surface-roughening treatment, an electrochemical surface-roughening treatment, and a chemical surface-roughening treatment may be appropriately used.

Meanwhile, it is known that an image quality of an ultrasound image depends on a difference in acoustic impedance of each medium through which an ultrasound wave is transmitted. At an interface in each medium, since the smaller the difference in acoustic impedance is, the more ultrasound waves are transmitted, the signal strength is increased and the image quality is improved, and since the larger the difference in acoustic impedance is, the more ultrasound waves are reflected, noise is increased and the image quality deteriorates.

As described above, in the image acquisition device 10, the ultrasound probe 30 irradiates the breast put into the compressed state by the compression member 40 with the ultrasound waves via the compression member 40 and receives the reflected waves from the breast. That is, the ultrasound wave is transmitted through an ultrasound radiation surface of the ultrasound probe 30, the bottom portion 43 of the compression member 40, and the breast or the like as a medium. In this case, a difference in acoustic impedance is generated at an interface between the ultrasound radiation surface of the ultrasound probe 30 and the upper surface 43A of the compression member 40 and at an interface between the contact surface 43B of the compression member 40 and the breast, which is a factor that increases noise and deteriorates the image quality.

Therefore, in the compression member 40 according to the present embodiment, the change in the acoustic impedance is smoothed by applying a gel-like or liquid-like medium having ultrasound transmittance (hereinafter, referred to as "echo jelly 4") to a portion of the uneven structure provided on the upper surface 43A and/or the contact surface 43B. That is, the image acquisition device 10 acquires the ultrasound image of the breast put into the compressed state by the compression member 40 in a state in which the echo jelly 4 is applied to the portion of the uneven structure, via the compression member 40.

As the echo jelly 4, for example, a known jelly for an ultrasound examination having acoustic impedance close to the acoustic impedance of the human body (breast) can be applied. Specifically, it is preferable that the echo jelly 4 has acoustic impedance between the acoustic impedance of the breast and the acoustic impedance of the compression member 40 (bottom portion 43). By filling the echo jelly 4 in a concave portion of the uneven structure, the acoustic impedance can be gradually changed. Therefore, a noise generated in accordance with the magnitude of the difference in acoustic impedance can be suppressed, so that the image quality of the ultrasound image can be improved.

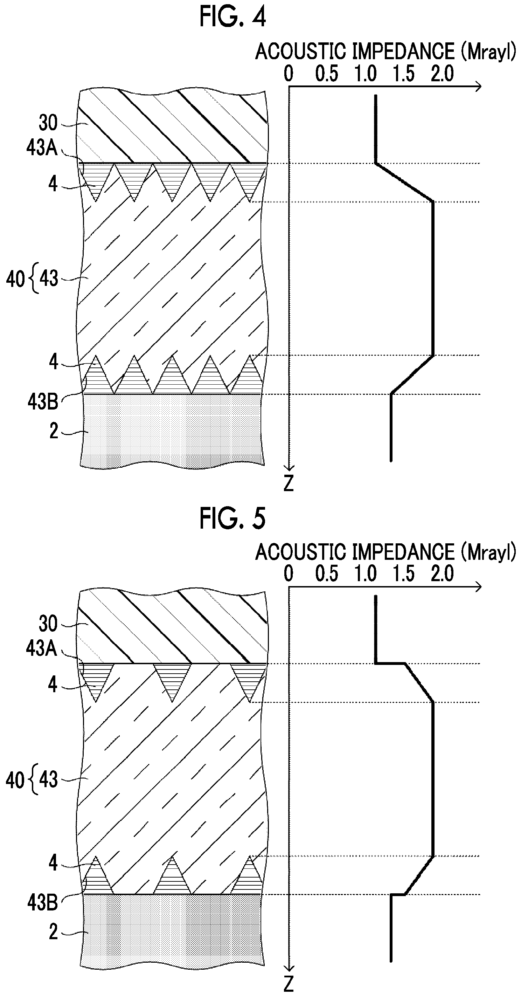

Figs. 4 to 9 show specific examples of the uneven structure. Views on the left side in Figs. 4 to 9 are enlarged cross-sectional views simulating a state in which the ultrasound probe 30 is in contact with the upper surface 43A side of the compression member 40, a breast 2 is in contact with the contact surface 43B side, and the echo jelly 4 is filled in the concave portion of the uneven structure provided on the upper surface 43A and/or the contact surface 43B. Views on the right side in Figs. 4 to 9 are graphs showing changes in acoustic impedance in the vertical direction (Z direction) corresponding to the enlarged cross-sectional views on the left side. In Figs. 4 to 9, a unit of the acoustic impedance is described as MRayl, in which 1 MRayl is 10⁶ kg/(m²·s).

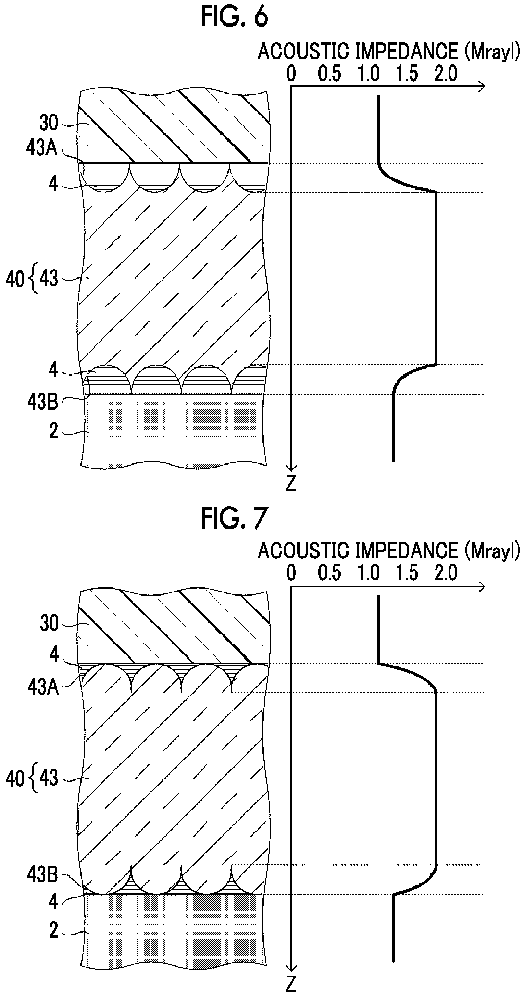

Figs. 4 and 5 show an example of a structure having a conical concave portion on the upper surface 43A and the contact surface 43B. In Fig. 5, a density of a concave portion is smaller than that in Fig. 4, and the average length RSm is longer than that in Fig. 4. Fig. 6 is an example of a structure having a hemispherical concave portion on the upper surface 43A and the contact surface 43B. Fig. 7 is an example of a structure having a concave portion formed by cutting out other portions while leaving the hemisphere, on the upper surface 43A and the contact surface 43B.

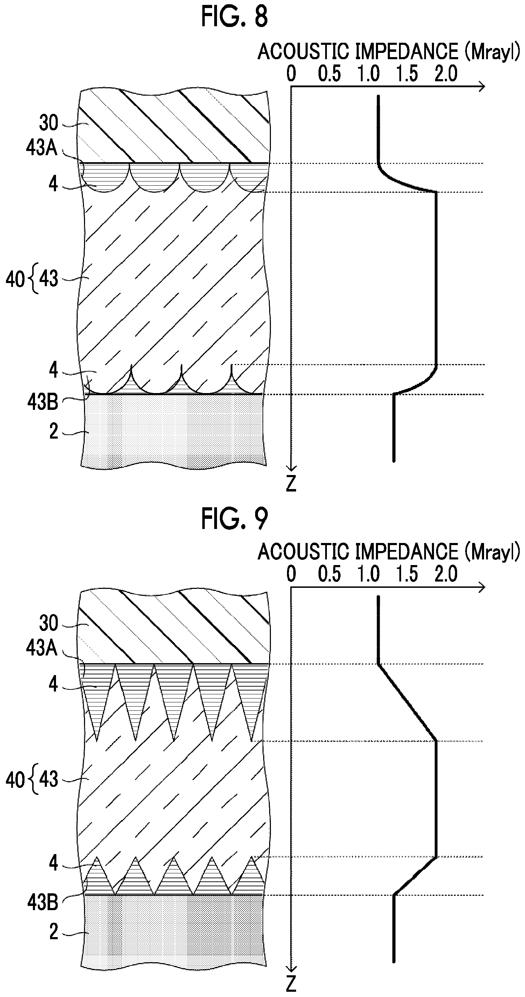

Fig. 8 is an example of a structure in which shapes of the uneven structures are different between the upper surface 43A and the contact surface 43B, the upper surface 43A has the hemispherical concave portion, and the contact surface 43B has the concave portion formed by cutting out other portions while leaving the hemisphere. Fig. 9 is an example of a structure having a conical concave portion with the maximum height roughness Rz different from each other on each of the upper surface 43A and the contact surface 43B.

As shown in Figs. 4 to 9, a manner of change in the acoustic impedance varies in accordance with the shape of unevenness, the maximum height roughness Rz, and the average length RSm of the uneven structure. Specifically, the acoustic impedance changes in proportion to a volume of the echo jelly 4. Therefore, for example, the shape of unevenness, the maximum height roughness Rz, and the average length RSm of the uneven structure may be appropriately optimized in accordance with the acoustic impedance, the thickness, and the like of the ultrasound probe 30, the compression member 40, the breast 2, and the echo jelly 4.

In addition, Figs. 4 to 9 show examples in which the uneven structure has a certain pattern, but the present disclosure is not limited thereto. The uneven structure may be randomly formed or may be formed in, for example, a wrinkle shape or a pear skin shape. In addition, each of the patterns shown in Figs. 4 to 9 may be combined.

As described above, the compression member 40 according to the present embodiment is a compression member that puts the breast into the compressed state, in which the uneven structure is provided in at least a part of a region on at least one of the contact surface 43B with the breast or the outer surface (upper surface 43A) facing the contact surface 43B, and the maximum height roughness Rz and the average length Rsm of the uneven structure are smaller than the wavelength of the ultrasound wave.

That is, with the compression member 40 according to the present embodiment, an appropriate amount of the echo jelly 4 can be held without affecting the ultrasound image. Therefore, the change in the acoustic impedance between the ultrasound probe 30 and the compression member 40 and between the compression member 40 and the breast 2 can be smoothed, and the noise generated in accordance with the magnitude of the difference in acoustic impedance can be suppressed, so that a favorable image can be obtained.

A method of imaging the breast by the image acquisition device 10 is not particularly limited. For example, cranio-caudal (CC) imaging, medio-lateral oblique (MLO) imaging, magnified imaging and spot imaging for imaging a part of the breast, and the like may be performed. The CC imaging is a method of imaging the breast in a compressed state by sandwiching the breast between the imaging table 16 and the compression member 40 in the vertical direction (Z direction). The MLO imaging is a method of imaging the breast in a compressed state including an axillary portion by sandwiching the breast between the imaging table 16 and the compression member 40 in a tilted state in which a rotation angle of the arm part 12 with respect to the base 14 is equal to or more than 45 degrees and less than 90 degrees.

Further, for example, the image acquisition device 10 may perform tomosynthesis imaging. In the tomosynthesis imaging, the radiation R is emitted from each of a plurality of irradiation positions having different irradiation angles toward the breast by the radiation source 17R, and a plurality of radiation images of the breast are captured. That is, in the tomosynthesis imaging, the imaging is performed by changing a rotation angle of the radiation emitting unit 17 with respect to the base 14 while keeping angles of the imaging table 16, the compression member 40, the breast, and the like.

In addition, in the image acquisition device 10, the breast of the subject may be positioned not only in a state in which the subject is standing up (standing state), but also in a state in which the subject is sitting on a chair, a wheelchair, or the like (sitting state).

Next, the console 50 will be described. The console 50 controls the image acquisition device 10 such that the radiation image is acquired in accordance with an imaging order acquired from the RIS 6, an instruction from the user, and the like. In addition, a position of the ultrasound probe 30 is controlled such that the ultrasound image can be acquired in accordance with a position of a region of interest included in the radiation image captured in the image acquisition device 10.

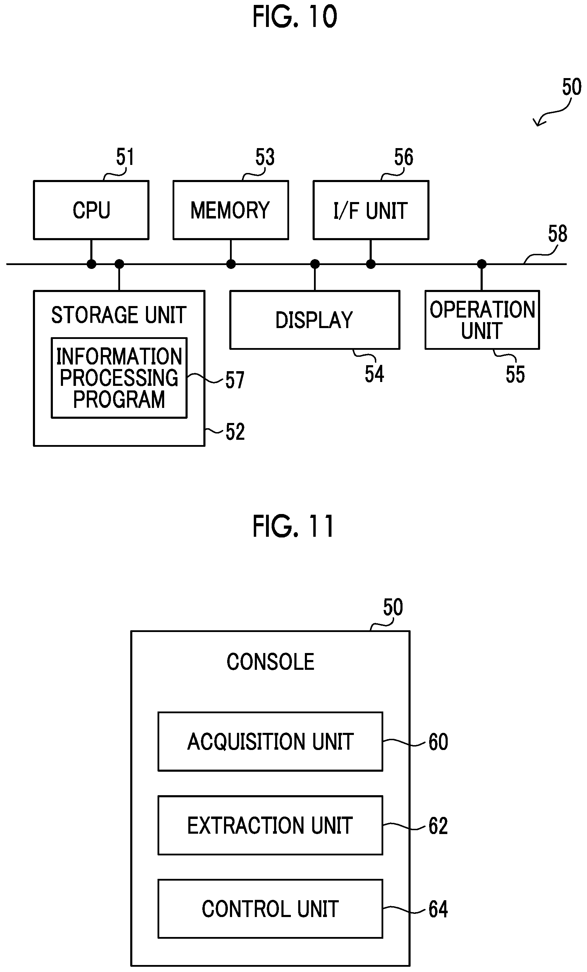

An example of a hardware configuration of the console 50 will be described with reference to Fig. 10. As shown in Fig. 10, the console 50 includes a CPU 51, a non-volatile storage unit 52, and a memory 53 as a temporary storage area. In addition, the console 50 includes a display 54 such as a liquid crystal display, an operation unit 55 such as a touch panel, a keyboard, and a mouse, and an I/F unit 56. The I/F unit 56 performs wired or wireless communication with the image acquisition device 10, the RIS 6, other external devices, and the like. The CPU 51, the storage unit 52, the memory 53, the display 54, the operation unit 55, and the I/F unit 56 are connected to each other via a bus 58 such as a system bus and a control bus such that various types of information can be exchanged.

The storage unit 52 is realized by, for example, a storage medium such as an HDD, an SSD, and a flash memory. The storage unit 52 stores an information processing program 57 in the console 50. The CPU 51 reads the information processing program 57 from the storage unit 52, develops the information processing program 57 into the memory 53, and executes the developed information processing program 57. As the console 50, for example, a personal computer, a server computer, a smartphone, a tablet terminal, a wearable terminal, or the like can be appropriately applied.

In addition, the storage unit 52 stores image data of the radiation image and the ultrasound image acquired by the image acquisition device 10, various types of other information, and the like. The image data of the radiation image and the ultrasound image may be stored in association with at least one of an imaging order or imaging information. The imaging information may include, for example, at least one of subject information and an imaging item that are included in the imaging order, photographer information indicating a photographer (for example, a user such as a doctor or a technician) who performed the imaging, or date and time information indicating date and time when the imaging was performed.

An example of a functional configuration of the console 50 will be described with reference to Fig. 11. As shown in Fig. 11, the console 50 includes an acquisition unit 60, an extraction unit 62, and a control unit 64. By executing the information processing program 57 by the CPU 51, the CPU 51 functions as the acquisition unit 60, the extraction unit 62, and the control unit 64.

### (Imaging instruction Based on Imaging Order)

The acquisition unit 60 acquires the imaging order from the RIS 6. The imaging order includes, for example, subject information such as a name, gender, and date of birth of the subject to be captured, an imaging item for performing the imaging, and the like. The imaging item is designated, for example, for each of the left and right breasts in various types of imaging, such as CC imaging, MLO imaging, magnified imaging, and spot imaging.

The control unit 64 gives an instruction to the image acquisition device 10 to capture the radiation image in accordance with the imaging order acquired by the acquisition unit 60.

### (Acquisition of Ultrasound Image)

After the radiation image is captured by the image acquisition device 10 in accordance with an imaging instruction from the control unit 64, the acquisition unit 60 acquires the radiation image. Specifically, the acquisition unit 60 may acquire the radiation image stored in the storage unit 22 of the image acquisition device 10 via the I/F unit 56, may acquire the radiation image stored in the storage unit 52, or may acquire the radiation image stored in the external device.

The extraction unit 62 extracts a region of interest included in the radiation image acquired by the acquisition unit 60. The region of interest is, for example, a region of abnormal shadow that appears on the radiation image due to a lesion such as a calcification or a tumor. The region of interest may be extracted using, for example, a known computer aided detection/diagnosis (CAD) technique, or a region designated by the user via the operation unit 55 may be extracted as the region of interest.

As a method of extracting the region of interest using the CAD technique, for example, a method using a learning model such as a convolutional neural network (CNN) may be applied. For example, the extraction unit 62 may extract the region of interest from the radiation image by using a learning model that is trained such that the radiation image is input and the region of interest included in the radiation image is extracted and output.

The control unit 64 controls the position of the ultrasound probe 30 in the image acquisition device 10 such that the region of interest extracted from the radiation image by the extraction unit 62 is reflected in the ultrasound image. Specifically, the control unit 64 specifies a position on the compression member 40 corresponding to the region of interest, and gives an instruction to the image acquisition device 10 to move the ultrasound probe 30 to the specified position on the compression member 40.

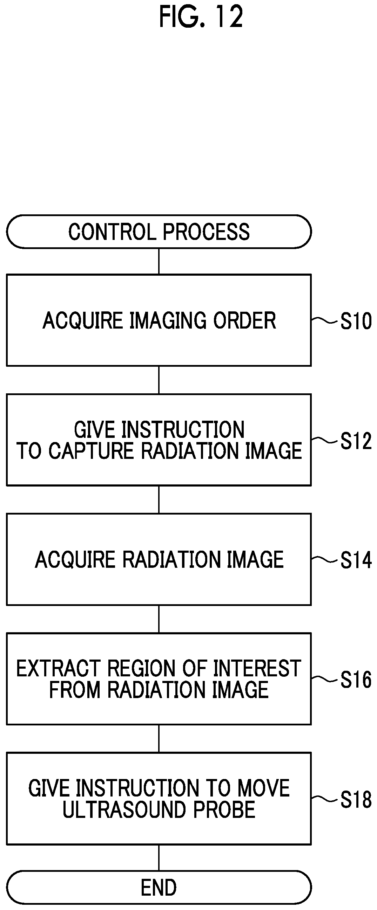

Next, a description of an action of the console 50 according to the present embodiment will be made with reference to Fig. 12. In the console 50, the CPU 51 executes the information processing program 57 to execute a control process shown in Fig. 12. The control process is executed, for example, in a case where the user gives an instruction to start execution via the operation unit 55.

In step S10, the acquisition unit 60 acquires the imaging order from the RIS 6. In step S12, the control unit 64 gives an instruction to the image acquisition device 10 to capture the radiation image in accordance with the imaging order acquired in step S10.

In step S14, the acquisition unit 60 acquires the radiation image captured by the image acquisition device 10 in accordance with the instruction in step S12. In step S16, the extraction unit 62 extracts the region of interest included in the radiation image acquired in step S14. In step S18, the control unit 64 gives an instruction to the image acquisition device 10 such that the ultrasound probe 30 is moved to a position in which the region of interest extracted in step S16 seems to be reflected in the ultrasound image. In a case where step S18 is completed, the present control process is terminated.

By controlling the image acquisition device 10 by the console 50 as described above, the image acquisition device 10 can acquire the ultrasound image including the region of interest in the radiation image.

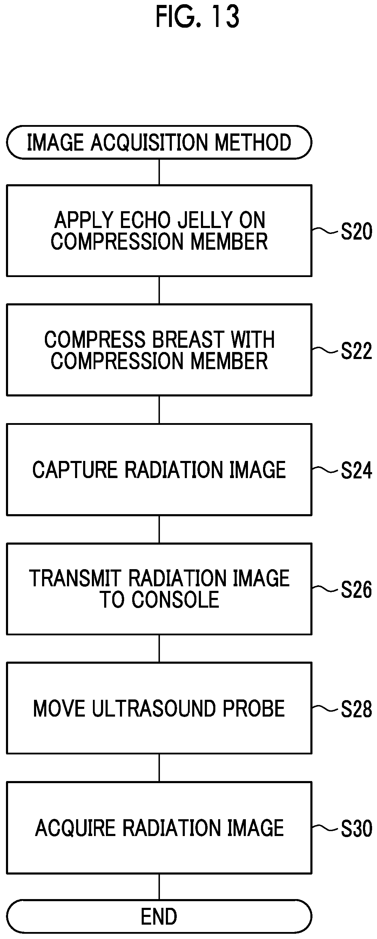

Next, with reference to Fig. 13, an image acquisition method of acquiring the radiation image and the ultrasound image using the image acquisition device 10, the console 50, and the compression member 40 according to the present embodiment will be described.

In step S20, the user applies the gel-like or liquid-like medium (echo jelly 4) having ultrasound transmittance to the portion of the uneven structure on the upper surface 43A and/or the contact surface 43B of the compression member 40. In step S22, the user performs the compression and the positioning of the breast using the compression member 40 in a state in which the echo jelly 4 is applied in step S20.

In step S24, the image acquisition device 10 irradiates the breast put into the compressed state in step S22 with the radiation R and captures the radiation image by the radiation detector 28. It should be noted that the capturing of the radiation image is performed in accordance with an instruction from the console 50 (corresponding to step S12). In step S26, the image acquisition device 10 transmits the radiation image captured in step S24 to the console 50.

In step S28, the image acquisition device 10 controls a driving unit of the probe unit 38 such that the ultrasound probe 30 is moved to a position in which the region of interest included in the radiation image captured in step S24 seems to be reflected in the ultrasound image. The ultrasound probe 30 is moved in accordance with the instruction from the console 50 (corresponding to step S18). In step S30, the image acquisition device 10 acquires the ultrasound image by the ultrasound probe 30 at the position moved in step S28. That is, the image acquisition device 10 acquires the ultrasound image of the breast put into the compressed state in step S22 by the compression member 40 in a state in which the echo jelly 4 is applied in step S20, via the compression member 40. In a case where step S30 is completed, the present image acquisition method is terminated.

### (Comparative Example)

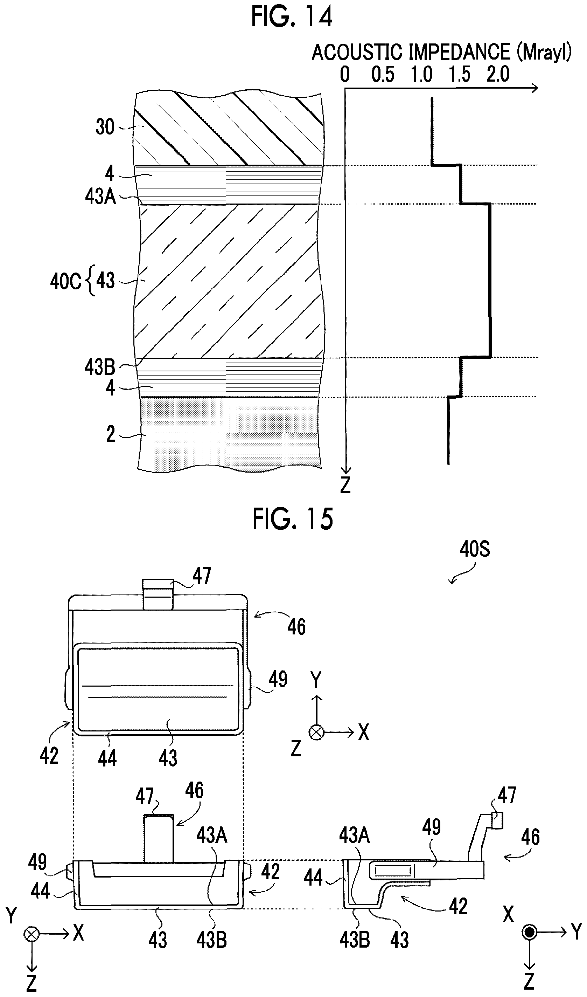

As a comparative example of the compression member 40 according to the present embodiment, Fig. 14 shows an example of a compression member 40C having no uneven structure. A view on the left side in Fig. 14 is an enlarged cross-sectional view simulating a state in which the ultrasound probe 30 is in contact with the upper surface 43A side of the compression member 40C, the breast 2 is in contact with the contact surface 43B side, and the echo jelly 4 is applied to the upper surface 43A and the contact surface 43B at a substantially constant thickness. A view on the right side in Fig. 14 is a graph showing a change in the acoustic impedance in the vertical direction (Z direction) corresponding to the enlarged cross-sectional view on the left side.

As shown in Fig. 14, in the compression member 40C that does not have the uneven structure, the acoustic impedance changes in a stepwise manner. Therefore, the noise generated in accordance with the magnitude of the difference in acoustic impedance between the ultrasound probe 30 and the echo jelly 4, between the echo jelly 4 and the compression member 40, and between the echo jelly 4 and the breast 2 is increased to be more than that of the compression member 40 according to the above-described embodiment. That is, the image quality of the ultrasound image deteriorates as compared with a case where the compression member 40 according to the above-described embodiment is used.

It should be noted that, in the above-described embodiment, a form is described in which the image acquisition device 10 comprises the ultrasound probe 30, but the present disclosure is not limited thereto. For example, the compression member 40 according to the present embodiment may be applied to a general mammography apparatus comprising the radiation source 17R, the radiation detector 28, and the compression member 40 disposed between the radiation source 17R and the radiation detector 28. Even in this case, for example, in a case where the ultrasound image of the breast put into the compressed state in the mammography apparatus is acquired by using the ultrasound probe independent of the mammography apparatus, a favorable image can be obtained.

In addition, in the above-described embodiment, a plurality of types of compression members 40 different from each other may be attachable to the image acquisition device 10. For example, the compression members 40 each having different types may be prepared in accordance with the physique (for example, the size of the breast) of the subject, the type of the imaging (for example, magnified imaging and spot imaging), or the like, and may be attachable to and detachable from the image acquisition device 10.

Specifically, a compression member in accordance with the size of the breast, a compression member for axilla imaging, a compression member for magnified imaging, and a compression member for so-called spot imaging that captures the radiation image of only a region in which a lesion exists, and the like may be used. That is, the compression member 40 is not limited to the one that compresses the entire breast, and may be smaller than the breast that seems to compress a part of the breast.

Fig. 15 shows a three-view of a compression member 40S for a small breast as an example of another embodiment of the compression member 40. The three-view of Fig. 15 includes a top view of the compression member 40S as viewed from above (radiation emitting unit 17 side), a side view thereof as viewed from the subject side, and a side view thereof as viewed from the right side of the subject. The compression member 40S includes the compression part 42 and the support part 46, similarly to the compression member 40 in Fig. 3. In the compression member 40S, the bottom portion 43 is not flat, and the attachment portion 47 side is higher than a chest wall side (side away from attachment portion 47). In addition, a height of the wall portion 44 is not uniform, and a height of a part of the chest wall side is lower than a height of other portions. Due to such a shape, the compression member 40S can easily perform positioning and compression even in a small breast.

In addition, in the above-described embodiment, for example, as hardware structures of processing units that execute various types of processes, such as the control unit 20, the acquisition unit 60, the extraction unit 62, and the control unit 64, various processors shown below can be used. The various types of processors include, as described above, a CPU which is a general-purpose processor that executes software (program) to function as various types of processing units, as well as a programmable logic device (PLD) which is a processor having a circuit configuration that can be changed after manufacturing such as a field programmable gate array (FPGA), a dedicated electrical circuit which is a processor having a circuit configuration specially designed to execute specific processing such as an application specific integrated circuit (ASIC), and the like.

One processing unit may be configured with one of the various types of processors, or a combination of two or more processors of the same type or different types (for example, a combination of a plurality of FPGAs, or a combination of a CPU and an FPGA). Further, a plurality of processing units may be configured with one processor.

As an example of configuring a plurality of processing units with one processor, first, there is a form in which, as typified by computers such as a client and a server, one processor is configured by combining one or more CPUs and software, and the processor functions as a plurality of processing units. Second, there is a form in which, as typified by a system on chip (SoC) and the like, a processor that implements functions of an entire system including a plurality of processing units with one integrated circuit (IC) chip is used. As described above, the various types of processing units are configured using one or more of the various types of processors as a hardware structure.

Furthermore, as the hardware structure of the various types of processors, more specifically, an electric circuitry in which circuit elements such as semiconductor elements are combined can be used.

In addition, in the above-described embodiments, an aspect has been described, in which various programs in the image acquisition device 10 are stored (installed) in advance in the ROM included in the control unit 20, and the information processing program 57 in the console 50 is stored in advance in the storage unit 52, but the present disclosure is not limited thereto. The various programs in the image acquisition device 10 and the information processing program 57 may be provided in a form recorded in a recording medium such as a compact disc read only memory (CD-ROM), a digital versatile disc read only memory (DVD-ROM), and a universal serial bus (USB) memory. In addition, the various programs in the image acquisition device 10 and the information processing program 57 may be provided in a form downloaded from an external device via a network. Furthermore, in addition to the program, the technique of the present disclosure extends to a storage medium that non-transitorily stores the program.

The technique of the present disclosure can also appropriately combine the above-described embodiments and examples. The above-described contents and illustrated contents are detailed descriptions of parts related to the technique of the present disclosure, and are merely examples of the technique of the present disclosure. For example, the above descriptions related to configurations, functions, operations, and effects are descriptions related to examples of configurations, functions, operations, and effects of the parts related to the technique of the present disclosure.

The disclosure of Japanese Patent Application No. 2022-054511 filed on March 29, 2022 is acknowledged.

## Claims

1. A compression member (40) configured to put a breast into a compressed state, comprising:
a contact surface (43B) with the breast; and
an outer surface (43A) facing the contact surface, wherein:
an uneven structure is provided in at least a part of a region on at least one of the contact surface or the outer surface (43A),
the compression member **characterized in that** the uneven structure has a maximum height roughness (Rz) and an average length (Rsm) of the uneven structure are equal to or less than 20 µm,
wherein the uneven structure of the outer surface (43A) and the contact surface (43B) are directed towards the interior of the compression member (40), and
wherein the uneven structure is provided in at least a part of a region on each of the contact surface (43B) and the outer surface (43A).

2. The compression member (40) according to claim 1, wherein the uneven structure is provided in at least a part of a region on at least the contact surface (43B).

3. The compression member (40) according to claim 1, wherein at least one of a shape of unevenness, a maximum height roughness (Rz), or an average length (Rsm) is different between the uneven structure provided on the contact surface (43B) and the uneven structure provided on the outer surface (43A).

4. An image acquisition system (1) comprising:
a radiation source (17R);
a radiation detector (28);
a compression member (40) configured to put a breast into a compressed state, which is disposed between the radiation source and the radiation detector, the compression member comprising:
a contact surface (43B) with the breast; and
an outer surface (43A) facing the contact surface, wherein:
an uneven structure is provided in at least a part of a region on at least one of the contact surface or the outer surface; and
the image acquisition system further comprising an ultrasound probe (30) disposed between the radiation source and the compression member.
the system **characterized in that** the uneven structure of the compression member has a maximum height roughness (Rz) and an average length (Rsm) of the uneven structure are equal to or less than 1/10 of a wavelength of an ultrasound wave emitted by the ultrasound probe,
wherein the uneven structure of the outer surface (43A) and the contact surface (43B) are directed towards the interior of the compression member (40), and
wherein the uneven structure is provided in at least a part of a region on each of the contact surface (43B) and the outer surface (43A).

5. The image acquisition system (1) according to claim 4, wherein the maximum height roughness (Rz) and the average length (Rsm) of the uneven structure are equal to or less than 1/20 of the wavelength of the ultrasound wave.

6. An image acquisition method comprising:
applying (S20) a gel-like or liquid-like medium (4) having ultrasound transmittance to a portion of an uneven structure in a compression member (40) that puts (S22) a breast into a compressed state; and
acquiring (S30) an ultrasound image of the breast in the compressed state by the compression member in a state in which the medium is applied, via the compression member,
wherein the compression member comprises:
a contact surface (43B) with the breast; and
an outer surface (43A) facing the contact surface, wherein:
the uneven structure is provided in at least a part of a region on at least one of the contact surface or the outer surface,
the method **characterized in that**
the uneven structure of the outer surface (43A) and the contact surface (43B) are directed towards the interior of the compression member (40), and
wherein the uneven structure is provided in at least a part of a region on each of the contact surface (43B) and the outer surface (43A).

7. The image acquisition method according to claim 6, wherein the medium (4) has acoustic impedance between acoustic impedance of the breast and acoustic impedance of the compression member (40).

8. The image acquisition method according to claim 6 or 7, wherein a concave portion of the uneven structure is filled with the medium (4).

## Patentansprüche

1. Kompressionselement (40), das so konfiguriert ist, dass es eine Brust in einen
komprimierten Zustand versetzt, umfassend:
eine Kontaktfläche (43B) mit der Brust; und
eine Außenfläche (43A), die der Kontaktfläche zugewandt ist, wobei:
eine ungleichmäßige Struktur in mindestens einem Teil eines Bereichs auf mindestens einer von der Kontaktfläche und der Außenfläche (43A) vorgesehen ist,
wobei das Kompressionselement **dadurch gekennzeichnet ist, dass** die ungleichmäßige Struktur eine maximale Höhenrauheit (Rz) und eine durchschnittliche Länge (Rsm) der ungleichmäßigen Struktur aufweist, die jeweils gleich oder kleiner als 20 µm sind,
wobei die ungleichmäßige Struktur der Außenfläche (43A) und der Kontaktfläche (43B) zu dem Inneren des Kompressionselements (40) hin gerichtet sind, und
wobei die ungleichmäßige Struktur in mindestens einem Teil eines Bereichs auf jeweils der Kontaktfläche (43B) und der Außenfläche (43A) vorgesehen ist.

2. Kompressionselement (40) nach Anspruch 1, wobei die ungleichmäßige Struktur in mindestens einem Teil eines Bereichs auf mindestens der Kontaktfläche (43B) vorgesehen ist.

3. Kompressionselement (40) nach Anspruch 1, wobei mindestens eines von einer Form von Unebenheit, einer maximalen Höhenrauheit (Rz) und einer durchschnittlichen Länge (Rsm) zwischen der auf der Kontaktfläche (43B) vorgesehenen ungleichmäßigen Struktur und der auf der Außenfläche (43A) vorgesehenen ungleichmäßigen Struktur unterschiedlich ist.

4. Bilderfassungssystem (1), umfassend:
eine Strahlungsquelle (17R);
einen Strahlungsdetektor (28);
ein Kompressionselement (40), das so konfiguriert ist, dass es eine Brust in einen komprimierten Zustand versetzt, das zwischen der Strahlungsquelle und dem Strahlungsdetektor angeordnet ist, wobei das Kompressionselement umfasst: eine Kontaktfläche (43B) mit der Brust; und
eine Außenfläche (43A), die der Kontaktfläche zugewandt ist, wobei:
eine ungleichmäßige Struktur in mindestens einem Teil eines Bereichs auf mindestens einer von der Kontaktfläche und der Außenfläche vorgesehen ist; und
das Bilderfassungssystem ferner eine Ultraschallsonde (30), die zwischen der Strahlungsquelle und dem Kompressionselement angeordnet ist, umfasst,
das System, **dadurch gekennzeichnet, dass** die ungleichmäßige Struktur des Kompressionselements eine maximale Höhenrauheit (Rz) und eine durchschnittliche Länge (Rsm) der ungleichmäßigen Struktur aufweist, die jeweils gleich oder kleiner als 1/10 einer Wellenlänge einer von der Ultraschallsonde ausgesendeten Ultraschallwelle sind,
wobei die ungleichmäßige Struktur der Außenfläche (43A) und der Kontaktfläche (43B) zu dem Inneren des Kompressionselements (40) hin gerichtet sind, und
wobei die ungleichmäßige Struktur in mindestens einem Teil eines Bereichs auf jeweils der Kontaktfläche (43B) und der Außenfläche (43A) vorgesehen ist.

5. Bilderfassungssystem (1) nach Anspruch 4, wobei die maximale Höhenrauheit (Rz) und die durchschnittliche Länge (Rsm) der ungleichmäßigen Struktur gleich oder kleiner als 1/20 der Wellenlänge der Ultraschallwelle sind.

6. Bilderfassungsverfahren, umfassend:
Aufbringen (S20) eines gelartigen oder flüssigkeitsartigen Mediums (4) mit Ultraschalltransmission auf einen Abschnitt einer ungleichmäßigen Struktur in einem Kompressionselement (40), das (S22) eine Brust in einen komprimierten Zustand versetzt; und
Erfassen (S30) eines Ultraschallbildes der Brust in dem komprimierten Zustand durch das Kompressionselement in einem Zustand, in dem das Medium aufgebracht ist, via das Kompressionselement,
wobei das Kompressionselement umfasst: eine
Kontaktfläche (43B) mit der Brust; und
eine Außenfläche (43A), die der Kontaktfläche zugewandt ist, wobei:
die ungleichmäßige Struktur in mindestens einem Teil eines Bereichs auf mindestens einer von der Kontaktfläche und der Außenfläche vorgesehen ist,
wobei das Verfahren **dadurch gekennzeichnet ist, dass**
die ungleichmäßige Struktur der Außenfläche (43A) und der Kontaktfläche (43B) zu dem Inneren des Kompressionselements (40) hin gerichtet sind, und
wobei die ungleichmäßige Struktur in mindestens einem Teil eines Bereichs auf jeweils der Kontaktfläche (43B) und der Außenfläche (43A) vorgesehen ist.

7. Bilderfassungsverfahren nach Anspruch 6, wobei das Medium (4) akustische Impedanz zwischen akustischer Impedanz der Brust und akustischer Impedanz des Kompressionselements (40) aufweist.

8. Bilderfassungsverfahren nach Anspruch 6 oder 7, wobei ein konkaver Abschnitt der ungleichmäßigen Struktur mit dem Medium (4) gefüllt ist.

## Revendications

1. Élément de compression (40) configuré pour mettre un sein dans un état comprimé, comprenant :
une surface de contact (43B) avec le sein ; et
une surface extérieure (43A) faisant face à la surface de contact, dans lequel :
une structure irrégulière est prévue dans au moins une partie d'une région sur au moins l'une de la surface de contact ou de la surface extérieure (43A),
l'élément de compression étant **caractérisé en ce que** la structure irrégulière présente une rugosité de hauteur maximale (Rz) et une longueur moyenne (Rsm) de la structure irrégulière égales ou inférieures à 20 µm,
dans lequel la structure irrégulière de la surface extérieure (43A) et de la surface de contact (43B) est orientée vers l'intérieur de l'élément de compression (40), et
dans lequel la structure irrégulière est prévue dans au moins une partie d'une région sur chacune de la surface de contact (43B) et de la surface extérieure (43A).

2. Élément de compression (40) selon la revendication 1, dans lequel la structure irrégulière est prévue dans au moins une partie d'une région sur au moins la surface de contact (43B).

3. Élément de compression (40) selon la revendication 1, dans lequel au moins l'une
parmi une forme d'irrégularité, une rugosité de hauteur maximale (Rz) et une longueur moyenne (Rsm) est différente entre la structure irrégulière prévue sur la surface de contact (43B) et la structure irrégulière prévue sur la surface extérieure (43A).

4. Système d'acquisition d'images (1) comprenant :
une source de rayonnement (17R) ;
un détecteur de rayonnement (28) ;
un élément de compression (40) configuré pour mettre un sein dans un état comprimé,
qui est disposé entre la source de rayonnement et le détecteur de rayonnement, l'élément de compression comprenant :
une surface de contact (43B) avec le sein ; et
une surface extérieure (43A) faisant face à la surface de contact, dans lequel :
une structure irrégulière est prévue dans au moins une partie d'une région sur au moins l'une de la surface de contact ou de la surface extérieure ; et
le système d'acquisition d'images comprenant en outre une sonde ultrasonore (30) disposée entre la source de rayonnement et l'élément de compression,
le système étant **caractérisé en ce que** la structure irrégulière de l'élément de compression présente une rugosité de hauteur maximale (Rz) et une longueur moyenne
(Rsm) de la structure irrégulière égales ou inférieures à 1/10 d'une longueur d'onde d'une onde ultrasonore émise par la sonde ultrasonore,
dans lequel la structure irrégulière de la surface extérieure (43A) et de la surface de contact (43B) est orientée vers l'intérieur de l'élément de compression (40), et
dans lequel la structure irrégulière est prévue dans au moins une partie d'une région sur chacune de la surface de contact (43B) et de la surface extérieure (43A).

5. Système d'acquisition d'images (1) selon la revendication 4, dans lequel la rugosité de
hauteur maximale (Rz) et la longueur moyenne (Rsm) de la structure irrégulière sont égales ou inférieures à 1/20 de la longueur d'onde de l'onde ultrasonore.

6. Procédé d'acquisition d'images comprenant :
appliquer (S20) un milieu de type gel ou liquide (4) ayant une transmittance
ultrasonore à une partie d'une structure irrégulière dans un élément de compression (40) qui met (S22) un sein dans un état comprimé ; et
acquérir (S30) une image ultrasonore du sein dans l'état comprimé par l'élément de compression dans un état dans lequel le milieu est appliqué, via l'élément de compression,
dans lequel l'élément de compression comprend :
une surface de contact (43B) avec le sein ; et
une surface extérieure (43A) faisant face à la surface de contact, dans lequel :
la structure irrégulière est prévue dans au moins une partie d'une région sur au moins l'une de la surface de contact ou de la surface extérieure,
le procédé étant **caractérisé en ce que**
la structure irrégulière de la surface extérieure (43A) et de la surface de contact (43B) est orientée vers l'intérieur de l'élément de compression (40), et
dans lequel la structure irrégulière est prévue dans au moins une partie d'une région sur chacune de la surface de contact (43B) et de la surface extérieure (43A).

7. Procédé d'acquisition d'images selon la revendication 6, dans lequel le milieu (4)
présente une impédance acoustique comprise entre l'impédance acoustique du sein et l'impédance acoustique de l'élément de compression (40).

8. Procédé d'acquisition d'images selon la revendication 6 ou la revendication 7, dans lequel une partie concave de la structure irrégulière est remplie du milieu (4).
